(19) Europäisches Patentamt
European Patent Office
Office européen des brevets

(11) Publication number: **0 572 530 B1**

# (12) EUROPEAN PATENT SPECIFICATION

(45) Date of publication of patent specification: **26.04.95**

(51) Int. Cl.[6]: **C07C 69/72**, C08F 8/00, C09D 133/06, C09D 167/00

(21) Application number: **92907185.0**

(22) Date of filing: **10.02.92**

(86) International application number: **PCT/US92/01038**

(87) International publication number: **WO 92/14694 (03.09.92 92/23)**

## (54) THERMOSETTING COATING COMPOSITIONS.

(30) Priority: **14.02.91 US 655480**
**31.07.91 US 737734**

(43) Date of publication of application: **08.12.93 Bulletin 93/49**

(45) Publication of the grant of the patent: **26.04.95 Bulletin 95/17**

(84) Designated Contracting States: **AT BE CH DE DK ES FR GB GR IT LI LU NL SE**

(56) References cited:
**EP-A- 0 227 454**
**EP-A- 0 376 859**

(73) Proprietor: **EASTMAN CHEMICAL COMPANY**
**100 North Eastman Road**
**Kingsport, TN 37660 (US)**

(72) Inventor: **BLOUNT, William, Williamson, Jr.**
**920 Broadwood Drive**
**Kingsport, TN 37660 (US)**
Inventor: **WITZEMAN, Jonathan, Stewart**
**1724 Lamount Street**
**Kingsport, TN 37664 (US)**

(74) Representative: **Behrens, Dieter, Dr.-Ing. et al**
**Wuesthoff & Wuesthoff**
**Patent- und Rechtsanwälte**
**Schweigerstrasse 2**
**D-81541 München (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention belongs to the field of organic chemistry. More particularly, it relates to a novel monomer compound and thermosetting coatings derived therefrom.

Considerable research effort continues in an effort to create surface coatings having superior handling properties, weatherability, impact resistance, chemical resistance, etc.

Among the more noteworthy thermosetting surface coating compositions are those based on melamine or melamine type cross-linkers. One critical drawback of such systems is that upon curing, formaldehyde is released into the atmosphere. The present invention thus provides certain compounds which have been found to serve as reactive diluents to increase the effective functionality of melamine-type cross-linkers in thermosetting surface coating compositions.

The present invention provides novel reactive diluents, dialkyl-propane-1,3-bis(acetoacetate) [$(Alk)_2$-$(AcAc)_2$], which are useful in thermosetting coating compositions. An especially preferred embodiment is butyl ethyl propane-1,3-bis(acetoacetate). Also provided are novel curable enamel compositions having this reactive diluent copolymerized therein. Surface coatings derived therefrom have been determined to possess outstanding acid resistance.

The present invention provides a compound of the Formula (A)

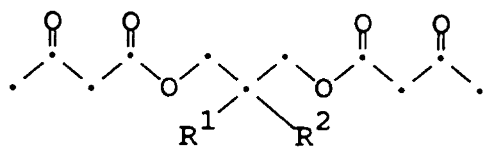

(A)

wherein $R^1$ and $R^2$ are individually $C_1$-$C_8$ alkyl, provided that when $R^1$ or $R^2$ is methyl, the other of $R^1$ or $R^2$ is $C_2$-$C_8$ alkyl.

The compound having Formula (A) is useful as a reactive diluent, i.e., as an adjunct to a cross-linking residue in polymeric coating formulations, in particular melamine based formulations. Coatings having residues of Formula (A) copolymerized therein have been demonstrated below to possess improved acid resistance. As an especially preferred embodiment of the present invention, $R^1$ is ethyl and $R^2$ is n-butyl.

The compound of Formula (A) can be prepared by reaction of a diol of the formula

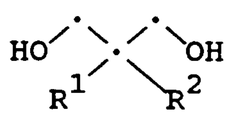

with diketene, with 2,2,6-trimethyl-4-H-1,3-dioxin-4-one (TKD), or by transacetoacetylation with acetoacetates such as t-butylacetoacetate, methylacetoacetate, or ethyl acetoacetate.

As noted above, the principal advantages of the compounds of Formula (A) involve their use as a reactive diluent or co-cross-linker. This application involves the use of the material to increase the effective functionality of the melamine cross-linker. This increase in effective functionality enables one to use less of the melamine cross-linker. If the main role of the compound of Formula (A) is to increase the effective functionality of the resin, rather than the cross-linker, it would be expected that more, not less, cross-linker would be required. It is thus believed that these materials are changing the mechanism of melamine cross-linking making it possible to use more of the available methylol groups on the melamine. As an additional beneficial consequence of the use of compounds of Formula (A) for partial replacement of melamine-type cross-linkers is the reduction of formaldehyde emission—a consequence of heat curing (i.e., cross-linking) of melamine-based thermosetting coatings. An added advantage of this approach is improvement in some of the coating properties, particularly acid resistance without reduction in other critical properties such as solvent resistance and impact strength. When compared to other bisacetoacetates used in this application, the coating having residues of Formula (A) copolymerized therein possessed better solvent properties and is outstanding in terms of improvement in acid resistance. In order for a material to be useful in this application it must both offer improved properties (i.e., acid resistance) and be of low enough volatility so that it is not lost during the thermosetting process. The material of the invention is less volatile than known materials such as neopentyl glycol bisacetoacetate and 2,2,4-trimethylpentan-1,3-diol bisacetoacetate.

Thus, as a further aspect of the present invention, there is provided a curable enamel composition comprising

(1) about 15 to about 80 weight percent of one or more curable polyesters and/or acrylic polymers;
(2) about 0 to about 50 weight percent of a suitable solvent;
(3) about 5 to about 40 weight percent of a cross-linking agent; and
(4) about 5 to about 40 weight percent of a compound of Formula (A)

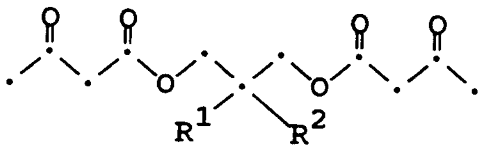

(A) .

It will be appreciated, of course, that all of the weight percentages above are based on the weight of the total composition (100%) and that when 1, 2, 3 and 4 are added, the sum must always equal 100%.

In the above composition, it is preferred that the polyester and/or acrylic component (1) be present in a range of about 30 to 70 weight percent, most preferably about 45 to 55 weight percent.

In the above composition, the solvent component (2) is preferably present in about 20 to about 50 weight percent, most preferably about 30 to 40 weight percent.

In the above composition, the cross-linking agent (component (3)) and the compound of Formula (A) (component (4)) are preferably present in about 5 to 25 weight percent, most preferably about 7.5 to 12.5 weight percent.

The polyester component (1) can be prepared by condensation polymerization methods known per se in the art. The most preferred method is to melt all reactants in a suitably sized reactor, heat the reactants to initiate the reaction, and continue processing until the desired molecular weight is reached. Reaction is evidenced by the collection of water (direct condensation) or alcohol (ester interchange). This procedure is referred to as fusion processing and can be conducted at atmospheric pressure or under vacuum. No modifications in these standard procedures are required for preparing suitable polymers for component (1), above.

In such curable polyesters, suitable diol and/or polyol residues are preferably selected from residues of ethylene glycol; propylene glycol; 1,3-propanediol; 2,4-dimethyl-2-ethylhexane-1,3-diol; 2,2-dimethyl-1,3-propanediol; 2-ethyl-2-butyl-1,3-propanediol; 2-ethyl-2-isobutyl-1,3-propanediol; 1,3-butanediol; 1,4-butanediol; 1,5-pentanediol, 1,6-hexanediol; 2,2,4-trimethyl-1,3-pentanediol; thiodiethanol; 1,2-cyclohexanedimethanol; 1,3-cyclohexanedimethanol; 1,4-cyclohexanedimethanol; 2,2,4,4-tetramethyl-1,3-cyclobutanediol; p-xylylenediol; diethylene glycol, triethylene glycol; tetraethylene glycol; and pentaethylene, hexaethylene, heptaethylene, octaethylene, nonaethylene, and decaethylene glycols.

Further, preferably the carboxylic acid residues of the curable polyesters are selected from residues of oxalic; malonic, dimethylmalonic; succinic; glutaric; adipic; trimethyladipic; pimelic, 2,2-dimethylglutaric; azelaic; sebacic, fumaric; maleic; itaconic; 1,3-cyclopentanedicarboxylic; 1,2-cyclohexanedicarboxylic; 1,3-cyclohexanedicarboxylic; 1,4-cyclohexanedicarboxylic; phthalic; terephthalic; isophthalic; 2,5-norbornanedicarboxylic; 1,4-naphthalic; diphenic; 4,4'-oxydibenzoic, diglycolic; thiodipropionic; 4,4'-sulfonyldibenzoic; and 2,6-naphthalenedicarboxylic acids.

Examples of commercially-available curable polyesters (component (1)) include Cargill 5770, Cargill 5722, and Aroplaz 6455 (Spencer Kellogg). In general, such polyesters will have hydroxyl values of about 20 to 200 (mg KOH/g polymer).

The acrylic polymer component (1) is preferably a polymer or resin prepared by polymerization of a hydroxyl-bearing monomer such as hydroxyethyl methacrylate, hydroxyethyl acrylate, hydroxyhexyl acrylate, hydroxyhexyl methacrylate, hydroxypropyl acrylate, hydroxypropyl methacrylate, hydroxybutyl acrylate, hydroxylbutyl methacrylate and the like optionally polymerized with other monomers such as methyl acrylate, methyl methacrylate, ethyl acrylate, ethyl methacrylate, butyl acrylate, butyl methacrylate, isobutyl acrylate, isobutyl methacrylate, ethylhexyl acrylate, ethylhexyl methacrylate, styrene, vinyl acetate, and the like. The ratio of reagents and molecular weights of the resulting acrylic polymer are preferably chosen so as to give polymers with an average functionality (the number of OH groups per molecule) greater than or equal to 2, preferably greater than or equal to 4.

Examples of commercially-available curable acrylic polymers include Joncryl 800, Joncryl 500, and Neocryl LE-800.

Suitable solvents for the curable enamel composition include xylenes, cyclohexanone, ketones, (for example, methyl amyl ketone), 2-butoxyethanol, ethyl-3-ethoxypropionate, toluene, and other volatile inert solvents typically used in industrial baking (i.e., thermosetting) enamels.

The cross-linking agent, component (3), above, is preferably a melamine-type cross-linking agent, i.e., a cross-linking agent having a plurality of $-N(CH_2OR)_2$ functional groups, wherein R is $C_1$-$C_4$ alkyl, preferably methyl.

In general, the cross-linking agent may be selected from compounds of the following formulae, wherein R is independently $C_1$-$C_4$ alkyl:

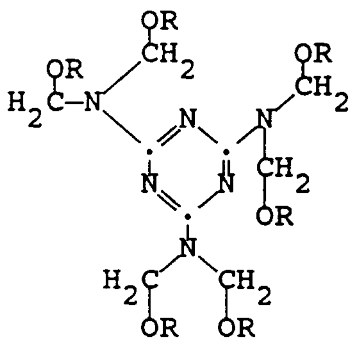 ,

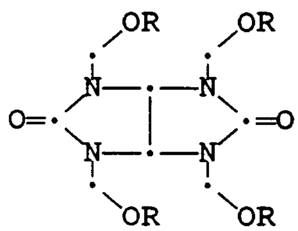 ,

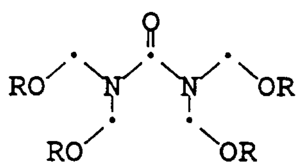 , or 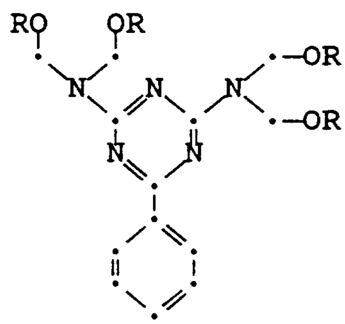 .

In this regard, preferred cross-linking agents include hexamethoxymethylmelamine, tetramethoxymethylbenzoguanamine, tetramethoxymethylurea, mixed butoxy/methoxy substituted melamines, and the like. The most preferred cross-linking agent is hexamethoxymethylmelamine.

As a further aspect of the present invention, there is provided a curable enamel composition further comprising one or more cross-linking catalysts. The most preferred cross-linking catalyst is p-toluene sulfonic acid.

As a further aspect of the present invention there is provided a cross-linkable enamel composition as described above, further comprising one or more leveling and flow control agents; pigment wetting and dispersing agents; cross-linking catalysts; tinting pigments; defoaming and antifoaming agents; anti-settling, anti-sag, and bodying agents; anti-skinning agents; anti-flooding and anti-floating agents; fungicides and mildewicides; thickening agents; or coalescing agents.

Such paint or coating additives as described above form a relatively minor proportion of the enamel composition, preferably about 0.05 weight % to about 5.00 weight %.

As a further aspect of the present invention, there is provided a curable enamel composition optionally containing one or more of the above-described additives, further comprising one or more pigments.

Pigments suitable for use in the enamel compositions envisioned by the present invention are the typical organic and inorganic pigments, well-known to one of ordinary skill in the art of surface coatings, especially those set forth by the Colour Index, 3d Ed., 2d Rev., 1982, published by the Society of Dyers and Colourists in association with the American Association of Textile Chemists and Colorists. Examples include, but are not limited to the following: CI Pigment White 6 (titanium dioxide); CI Pigment Red 101 (red iron oxide); CI Pigment Yellow 42, CI Pigment Blue 15, 15:1, 15:2, 15:3, 15:4 (copper phthalocyanines); CI Pigment Red 49:1; and CI Pigment Red 57:1.

Upon formulation above, the curable enamel composition is then applied to the desired substrate or article, e.g., steel, aluminum, or galvanized sheeting (either primed or unprimed), heated (i.e., cured) to a temperature of about 140 °C to about 175 °C, for a time period of 5-45 minutes and subsequently allowed to cool. Thus, as a further aspect of the present invention, there is provided a shaped or formed article which has been coated with the thermosetting coating compositions of the present invention and cured.

Further examples of typical application and curing methods can be found in U.S. Patent Nos. 4,737,551 and 4,698,391.

Thus, as a further aspect of the present invention, there is provided a coating which results from the application and curing of the curable enamel composition as set forth above.

$^{1}H$ NMR spectra below were obtained on a Varian Model Gemini 300 in $CDCl_3$ solvent at a frequency of 300 MHz. $^{13}C$ NMR spectra were obtained on the same instrument at a frequency of 75 MHz. Mass spectra were obtained on a VG model 7070VSEQ mass spectrometer.

The following abbreviations are used in the examples:

| | |
|---|---|
| $NPG(AcAc)_2$ * | neopentylglycol bis(acetoacetate) |
| $TMPD(AcAc)_2$ * | 2,2,4-trimethyl pentan-1,3-diol bis(acetoacetate) |
| $HD(AcAc)_2$ * | 1,6-hexandiol-bis(acetoacetate) |
| $EG(AcAc)_2$ * | ethylene glycol-bis(acetoacetate) |
| $BEPD(AcAc)_2$ | butyl ethyl propanediol bis(acetoacetate) |

* (these compounds are described in EP-A-376 859 and EP-A-227 454)

The applicable tests procedures as described herein are as follows:

1. Acid Value of resins (ASTM Method D 465)
2. Testing Coated Metal Specimens at 100 Percent Relative Humidity—Cleveland humidity (ASTM Method D 2247)
3. Ford Cup Viscosity (ASTM Method D 1200)
4. Molecular Weight (Gel Permeation Chromatography)
5. Gardner—Holdt Bubble Viscosity (ASTM Method D 1545)
6. Film Thickness (General Electric Gage, Type B)
7. Film Hardness (Pencil Method)
8. Solvent resistance (Methylethyl ketone (MEK) dynamic rub test (ASTM Method D 1308)
9. Staining Tests (ASTM Method D 1540)
10. Specular Gloss (ASTM Method D 523)
11. Hegmann Fineness—of—Dispersion (ASTM Method D 1210)
12. I.C.I. Cone and Plate Viscosity (ASTM Method D 4287)
13. Standard Method for Calculation of Color Differences From Instrumentally Measured Color Coordinates (ASTM Method D 2244)

The acrylic polymer used (Joncryl 800) was purchased from S. C. Johnson and Son Inc. It has a hydroxyl value of 47 and an acid number of 15. This material was used as a 55% solids solution in methyl amyl ketone.

EXAMPLE 1 - Preparation of Butyl Ethyl Propane-1,3-Bis Acetoacetate

In a 1 L, 3-necked flask equipped with magnetic stirrer, heating mantle, thermocouple temperature regulator and heating mantle and a Vigreux distillation column with still head, nitrogen inlet and thermometer was placed 217.99 g (1.36 mol) butyl ethyl propane-1,3-diol and 441.28 g (2.70 mol) t-butyl acetoacetate. The solution was heated and the t-butanol distillate collected. The reaction was stopped once the base temperature had reached 200 °C. A total of 250 mL (93% of theoretical) condensate had been collected which was shown by gas chromatography to be 99% tBuOH. The resulting crude material was vacuum stripped by passing it through a wiped-film still at 1.0 mm Hg with a wall temperature of 135 °C.

For 1: $^1H$ NMR: 0.83 (t, J = 7.5 Hz, 3H), 0.90 (t, J = 6.9 Hz, 3H), 1.12-1.38 (m, 8H), 2.26 (s, 6H), 3.47 (s, 4H), 3.97 (s, 4H). $^{13}C$ NMR: 6.51 ($CH_3$), 13.46 ($CH_3$), 22.85 ($CH_2$), 22.97 ($CH_2$), 24.14 ($CH_2$), 29.66 ($CH_2$), 29.70 ($CH_3$), 39.03 (C), 49.53 ($CH_2$), 66.28 ($CH_2$), 167.03 (C), 200.56 (C). IR (neat) 2953, 2927, 2862, 1740, 1718, 1465, 1408, 1359, 1318, 1239, 1149, 1031 $cm^{-1}$. High Resolution Mass Spectrum MW = 328.18864 (Calculated for $C_{18}H_{28}O_6$: 328.18859).

Reference Example 1 - Preparation of HS-3-1NCp Resin

A two-stage procedure was used to prepare the polyester resin identified as HS-3-1NCp. The following materials were first charged into a 2000-mL, three-necked round bottom flask fitted with a stirrer, nitrogen sparge tube (0.4 scfh), a 1/4-inch steel mesh packed partial condenser fitted with a Barett condensate trap and total condenser:

334.0 g (3.21 mol) 2,2-dimethyl-1,3-propanediol
67.8 g (0.51 mol) 1,1,1-trimethylolpropane
284.2 g (1.65 mol) 1,4-cyclohexanedicarboxylic acid
166.0 g (1.12 mol) phthalic anhydride
1.0 g butyl stannoic acid catalyst

The reaction mixture was heated at about 3°C/min to 140°C where the slurry can be stirred. Upheat was continued at about 1°C/min to 180°C where condensation polymerization began. The temperature was raised continuously to about 210°C to promote a constant rate of condensate collection in the Barett tube. When the reaction mixture clears and the acid value (mg KOH/g polymer) is less than 20 (approximately 4 hours processing time with the accumulation of 95 grams water), the mixture was then cooled to 150°C and the remaining reactants added:

67.8 g (0.51 mol) 1,1,1-trimethylolpropane
122.0 g (0.71 mol) 1,4-cyclohexanedicarboxylic acid
71.0 g (0.48 mol) phthalic anhydride

The mixture was heated at 1°C/min to 200°C to continue the polymerization. The reaction was considered complete when the acid value drops to below 10; this required another 3 hours' processing with accumulation of 42 additional grams of condensate. During the last hour, the nitrogen sparge was increased to about 2 scfh in order to aid the removal of water from the viscosity-increasing product. The polymer was cooled to 150°C, diluted to 75% non-volatiles with xylene, removed from the reactor to be converted into a finished coating formulation. It had a number average molecular weight of 2100, a weight average molecular weight of 7000, an acid value of 6, and a hydroxyl value of 108.

Example 2 - Comparison of Solvent Properties of Various Acetoacetates

This example illustrates the solvent properties of compound 1. A resin grind was prepared from 307.7 g resin from Reference Example 1, 282.6 g R-900 $TiO_2$ pigment and 45 g of a 60:20:20 solvent blend of methyl amyl ketone (MAK), ethyl ethoxy propionate (EEP) and n-butanol (n-BuOH). The following formulations were then prepared using this grind:

| | Example # | | | | | |
|---|---|---|---|---|---|---|
| | C-1 | C-2 | C-3 | C-4 | C-5 | 2 |
| Grind | 89.2 | 89.2 | 89.2 | 89.2 | 89.2 | 89.2 |
| Additional Resin (Reference Ex. 1) | 2.5 | 2.5 | 1.7 | 2.1 | 2.1 | 1.3 |
| Cymel 303 | 13.2 | 8.8 | 8.6 | 8.7 | 8.7 | 8.5 |
| $NPG(AcAc)_2$ | | | | | 4.8 | |
| $TMPD(AcAc)_2$ | | | 5.3 | | | |
| $TMP(AcAc)_3$ | | 4.4 | | | | |
| $HD(AcAc)_2$ | | | | 4.8 | | |
| $BEPD(AcAc)_2$ | | | | | | 5.6 |
| 40% pTSA (Catalyst) | 0.6 | 0.6 | 0.6 | 0.6 | 0.6 | 0.6 |
| 20% FC-430 | 0.1 | 0.1 | 0.1 | 0.1 | 0.1 | 0.1 |

The following solution viscosities were obtained for the above formulations:

| | Formulation # | | | | | |
|---|---|---|---|---|---|---|
| | C-1 | C-2 | C-3 | C-4 | C-5 | 2 |
| Solution Viscosity (centapoise) | 770 | 822 | 797 | 660 | 753 | 695 |

Example 3 - Evaluation of Butyl Ethyl Propane-1,3-Bis(Acetoacetate) in Coatings

This example illustrates that use of the compound of Formula (1) improves the acid resistance relative to Example C-1 without the loss in impact strength and hardness noted in Examples C-7 and C-8.

The following formulations were prepared:

| | Formulation # | | |
|---|---|---|---|
| | C-7 | C-8 | 3 |
| Grind from Example 2 | 91.3 | 91.3 | 89.2 |
| Additional Resin | 8.6 | 11.6 | 0.7 |
| Cymel 303 | 8.8 | 6.6 | 6.3 |
| $BEPD(AcAc)_2$ | | | 8.3 |
| 40% pTSA (Catalyst) | 0.6 | 0.6 | 0.6 |
| 20% FC-430 Flow Aid | 0.2 | 0.2 | 0.2 |

Formulations C-1, C-7, C-8 and 2 were then drawn down with #20 wire-wound rod on a 20 gauge phosphated steel and cured at 160 °C for 20 min. The following properties were noted:

| | Formulation # | | | | |
|---|---|---|---|---|---|
| | C-1 | C-7 | C-8 | 2 | 3 |
| Impact (Front/Reverse) | 120/20 | 50/<10 | 40/<10 | 80/10 | 160/10 |
| Pencil Hardness (Mar/cut) | 5H/6H | 2H/5H | 2H/5H | 5H/6H | 4H/5H |
| MEK Resistance | >250 | >250 | >250 | >250 | >250 |
| Iodine (5/30 min) | 5/4 | 5/5 | 4/3 | 4/2 | 3/1 |
| Mustard (C/U, 24 hr) | 5/5 | 5/5 | 5/5 | 5/5 | 5/5 |
| 50% NaOH (C/U, 24 hr) | 5/5 | 5/5 | 5/5 | 5/5 | 5/5 |
| 50% $H_2SO_4$ (C/U, 24 hr) | 3/3 | 5/5 | 5/5 | 4/5 | 4/5 |

Example 4

This example illustrates the use of butyl ethyl propane-1,3-bisacetoacetate in acrylic formulations.

A grind was prepared from 190.2 of Joncryl 800 as a 55% solids solution and 100.0 g $TiO_2$ and 15.0 g solvent blend. The following formulations were then prepared:

| | Formulation # | |
|---|---|---|
| | C-9 | 4 |
| Grind (from above) | 147.65 | 147.65 |
| Joncryl 800 (55% solids) | 15.52 | - |
| Cymel 303 | 4.35 | 2.18 |
| $BEPD(AcAc)_2$ | - | 6.36 |
| 40% pTSA | 0.69 | 0.69 |

The formulations were drawn down on metal panels and cured as before. The following properties were obtained for the resulting formulations:

| | Formulation # | |
|---|---|---|
| | C-9 | 4 |
| Impact (Front/reverse) | 60/<10 | 40/<10 |
| Pencil Hardness (Mar/cut) | F/H | 3H/4H |
| Iodine (5/30 min) | 4/2 | 4/2 |
| Mustard (C/U 24 hr) | 5/5 | 5/5 |
| 50% NaOH (C/U 24 hr) | 5/5 | 5/5 |
| 50% $H_2SO_4$ (C/U 24 hr) | 1/1 | 4/5 |

The difference in acid resistance is particularly dramatic in this example. Formulation C-9 shows complete loss of the coating upon exposure to 50% sulfuric while formulation 4 shows little effect what so ever.

Example 5 - Comparison of Volatility of Various Bisacetoacetates

The bisacetoacetates of ethylene glycol, BEPD, NPG, TMPD and 1,6-hexanediol were analyzed by thermogravimetric analysis by heating the materials from 25°C to 275°C at a rate of 10°C/min and noting the extent weight loss versus time. The following results were obtained:

| Bis-Acetoacetate | T (°C) for 50% Weight Loss | T (°C) for 100% Weight Loss |
|---|---|---|
| $EG(AcAc)_2$ | 230 | 260 |
| $TMPD(AcAc)_2$ | 220 | 235 |
| $NPG(AcAc)_2$ | 220 | 240 |
| $HD(AcAc)_2$ | 265 | >275 |
| $BEPD(AcAc)_2$ | 260 | >275 |

## Claims

**1.** A compound of the formula

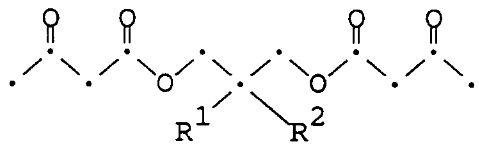

,

wherein $R^1$ and $R^2$ are individually $C_1$-$C_8$ alkyl, provided that when $R^1$ or $R^2$ is methyl, the other of $R^1$ or $R^2$ is $C_2$-$C_8$ alkyl.

**2.** The compound of Claim 1 wherein $R^1$ is ethyl and $R^2$ is n-butyl.

3. A curable enamel composition comprising
(1) 15 to 80 weight percent of one or more curable polyesters and/or acrylic polymers;
(2) 0 to 50 weight percent of a solvent;
(3) 5 to 40 weight percent of a cross-linking agent; and
(4) 5 to 40 weight percent of a compound of the formula

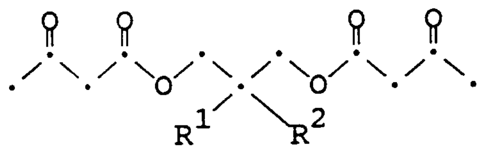
,

wherein $R^1$ and $R^2$ are individually $C_1$-$C_8$ alkyl, provided that when $R^1$ or $R^2$ is methyl, the other of $R^1$ or $R^2$ is $C_2$-$C_8$ alkyl.

4. The curable enamel composition of Claim 3 wherein the cross-linking agent has two or more groups of the formula $-N\{CH_2OCR)_2$, wherein R is $C_1$-$C_4$ alkyl.

5. The curable enamel composition of Claim 3 wherein the cross-linking agent is selected from hexamethoxymethylmelamine, tetramethoxymethylbenzoguanamine, tetramethoxymethylurea, or a mixed butoxyl/methoxy substituted melamine.

6. The curable enamel composition of Claim 3 wherein the cross-linking agent is hexamethoxymethylmelamine.

7. The curable enamel composition of Claim 3 wherein component (1) consists of one or more curable polyesters.

8. The curable enamel composition of Claim 3 wherein $R^1$ is ethyl and $R^2$ is n-butyl.

9. A curable enamel composition comprising
(1) 30 to 70 weight percent of one or more curable polyesters and/or acrylic polymers;
(2) 20 to 50 weight percent of a solvent;
(3) 5 to 25 weight percent of a cross-linking agent; and
(4) 5 to 25 weight percent of a compound of the formula

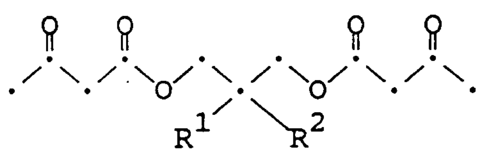
,

wherein $R^1$ and $R^2$ are individually $C_1$-$C_8$ alkyl, provided that when $R^1$ or $R^2$ is methyl, the other of $R^1$ or $R^2$ is $C_2$-$C_8$ alkyl.

10. The curable enamel composition of Claim 9 wherein the cross-linking agent has two or more groups of the formula $-N-(CH_2OR)_2$, wherein R is $C_1$-$C_4$ alkyl.

11. The curable enamel composition of Claim 9 or 10 wherein the cross-linking agent is selected from hexamethoxymethylmelamine, tetramethoxymethylbenzoguanamine, tetramethoxymethylurea, or a mixed butoxyl/methoxy substituted melamine.

12. The curable enamel composition of Claim 9 or 11 wherein the cross-linking agent is hexamethoxymethylmelamine.

13. The curable enamel composition of Claim 9, wherein component (1) consists of one or more curable polyesters.

14. The curable enamel composition of Claim 9 or 13 wherein $R^1$ is ethyl and $R^2$ is n-butyl.

15. A curable enamel composition comprising
(1) 45 to 55 weight percent of one or more curable polyesters and/or acrylic polymers;
(2) 30 to 40 weight percent of a solvent;
(3) 7.5 to 12.5 weight percent of a cross-linking agent; and
(4) 7.5 to 12.5 weight percent of a compound of the formula

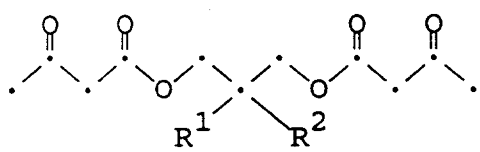
,

wherein $R^1$ and $R^2$ are individually $C_1$-$C_8$ alkyl, provided that when $R^1$ or $R^2$ is methyl, the other of $R^1$ or $R^2$ is $C_2$-$C_8$ alkyl.

16. The curable enamel composition of Claim 15 wherein the cross-linking agent has two or more groups of the formula $-N(CH_2OR)_2$, wherein R is $C_1$-$C_4$ alkyl.

17. The curable enamel composition of Claim 15 or 16 wherein the cross-linking agent is selected from hexamethoxymethylmelamine, tetramethoxymethylbenzoguanamine, tetramethoxymethylurea, or a mixed butoxy/methoxy substituted melamine.

18. The curable enamel composition of Claim 15 wherein the cross-linking agent is hexamethoxymethylmelamine.

19. The curable enamel composition of Claim 15 wherein $R^1$ is ethyl and $R^2$ is n-butyl, and component (1) consists of one or more curable polyesters.

20. The cross-linking enamel composition of Claim 3, 9 or 15 further comprising one or more leveling and flow control agents; pigment wetting and dispersing agents; cross-linking catalysts; tinting pigments; defoaming and antifoaming agents; anti-settling, anti-sag, and bodying agents; anti-skinning agents; anti-flooding and anti-floating agents; fungicides and mildewicides; thickening agents; or coalescing agents.

21. Application of the curable enamel compositions according to Claim 20 to a formed or shaped substrate and subsequent curing in order to obtain a coated or shaped article.

**Patentansprüche**

1. Verbindung der Formel

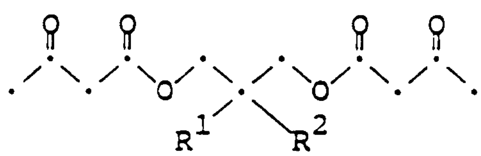
,

worin $R^1$ und $R^2$ individuell $C_1$-$C_8$-Alkyl sind mit der Maßgabe, daß, wenn $R^1$ oder $R^2$ Methyl ist, das andere $R^1$ oder $R^2$ $C_2$-$C_8$-Alkyl ist.

2. Verbindung nach Anspruch 1, worin $R^1$ Ethyl und $R^2$ n-Butyl ist.

**3.** Härtbare Schutzschicht-Zusammensetzung umfassend
(1) 15 bis 80 Gew.-% eines oder mehrerer härtbarer Polyester und/oder acrylischer Polymere;
(2) 0 bis 50 Gew.-% Lösungsmittel;
(3) 5 bis 40 Gew.-% eines Quervernetzungsmittels und
(4) 5 bis 40 Gew.-% einer Verbindung der Formel

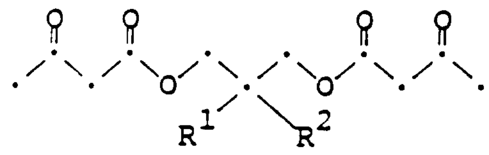
,

worin $R^1$ und $R^2$ individuell $C_1$-$C_8$-Alkyl sind mit der Maßgabe, daß, wenn $R^1$ oder $R^2$ Methyl ist, das andere $R^1$ oder $R^2$ $C_2$-$C_8$-Alkyl ist.

**4.** Härtbare Schutzschicht-Zusammensetzung nach Anspruch 3, wobei das Quervernetzungsmittel zwei oder mehr Gruppen der Formel $-N-(CH_2OCR)_2$ hat, wobei R $C_1$-$C_4$-Alkyl ist.

**5.** Härtbare Schutzschicht-Zusammensetzung nach Anspruch 3, wobei das Quervernetzungsmittel ausgewählt ist aus Hexamethoxymethylmelamin, Tetramethoxymethylbenzoguanamin, Tetramethoxymethyl-Harnstoff oder gemischt Butoxy/Methoxy-substituiertes Melamin.

**6.** Härtbare Schutzschicht-Zusammensetzung nach Anspruch 3, wobei das Quervernetzungsmittel Hexamethoxymethylmelamin ist.

**7.** Härtbare Schutzschicht-Zusammensetzung nach Anspruch 3, wobei der Bestandteil (1) aus einem oder mehreren härtbaren Polyestern besteht.

**8.** Härtbare Schutzschicht-Zusammensetzung nach Anspruch 3, wobei $R^1$ Ethyl und $R^2$ n-Butyl ist.

**9.** Härtbare Schutzschicht-Zusammensetzung umfassend
(1) 30 bis 70 Gew.-% eines oder mehrerer härtbarer Polyester und/oder acrylischer Polymere;
(2) 20 bis 50 Gew.-% Lösungsmittel;
(3) 5 bis 25 Gew.-% eines Quervernetzungsmittels und
(4) 5 bis 25 Gew.-% einer Verbindung der Formel

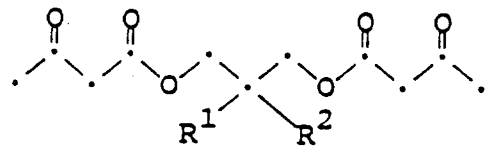
,

worin $R^1$ und $R^2$ individuell $C_1$-$C_8$-Alkyl sind mit der Maßgabe, daß, wenn $R^1$ oder $R^2$ Methyl ist, das andere $R^1$ oder $R^2$ $C_2$-$C_8$-Alkyl ist.

**10.** Härtbare Schutzschicht-Zusammensetzung nach Anspruch 9, wobei das Quervernetzungsmittel zwei oder mehrere Gruppen der Formel $-N-(CH_2OR)_2$ hat, wobei R $C_1$-$C_4$-Alkyl ist.

**11.** Härtbare Schutzschicht-Zusammensetzung nach Anspruch 9 oder 10, wobei das Quervernetzungsmittel ausgewählt ist aus: Hexamethoxymethylmelamin, Tetramethoxymethylbenzoguanamin, Tetramethoxymethyl-Harnstoff oder gemischt Butoxy/Methoxy-substituiertes Melamin.

**12.** Härtbare Schutzschicht-Zusammensetzung nach Anspruch 9 oder 11, wobei das Quervernetzungsmittel Hexamethoxymethylmelamin ist.

**13.** Härtbare Schutzschicht-Zusammensetzung nach Anspruch 9, wobei der Bestandteil (1) aus einem oder mehreren härtbaren Polyestern besteht.

**14.** Härtbare Schutzschicht-Zusammensetzung nach Anspruch 9 oder 13, wobei $R^1$ Ethyl und $R^2$ n-Butyl ist.

**15.** Härtbare Schutzschicht-Zusammensetzung umfassend
(1) 45 bis 55 Gew.-% eines oder mehrerer härtbarer Polyester und/oder acrylischer Polymere;
(2) 30 bis 40 Gew.-% Lösungsmittel;
(3) 7,5 bis 12,5 Gew.-% eines Quervernetzungsmittels und
(4) 7,5 bis 12,5 Gew.-% einer Verbindung der Formel

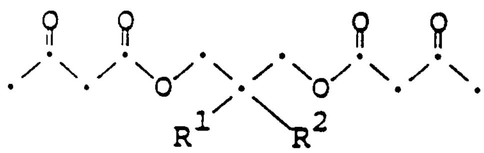,

worin $R^1$ und $R^2$ individuell $C_1$-$C_8$-Alkyl sind mit der Maßgabe, daß, wenn $R^1$ oder $R^2$ Methyl ist, das andere $R^1$ oder $R^2$ $C_2$-$C_8$-Alkyl ist.

**16.** Härtbare Schutzschicht-Zusammensetzung nach Anspruch 15, wobei das Quervernetzungsmittel zwei oder mehrere Gruppen der Formel $-N(CH_2OR)_2$ hat, worin R $C_1$-$C_4$-Alkyl ist.

**17.** Härtbare Schutzschicht-Zusammensetzunug nach Anspruch 15 oder 16, wobei das Vernetzungsmittel ausgewählt ist aus: Hexamethoxymethylmelamin, Tetramethoxymethylbenzoguanamin, Tetramethoxymethyl-Harnstoff oder gemischt Butoxy/Methoxy-substituiertes Melamin.

**18.** Härtbare Schutzschicht-Zusammensetzung nach Anspruch 15, wobei das Quervernetzungsmittel Hexamethoxymethylmelamin ist.

**19.** Härtbare Schutzschicht-Zusammensetzung nach Anspruch 15, wobei $R^1$ Ethyl ist und $R^2$ n-Butyl ist und der Bestandteil (1) aus einem oder mehreren härtbaren Polyestern besteht.

**20.** Quervernetzende Schutzschicht-Zusammensetzung nach Anspruch 3, 9 oder 15, die weiterhin eines oder mehrere Egalisierungs- und Verlaufmittel, Pigment-Befeuchtungs- und Dispergierungsmittel; Quervernetzungskatalysatoren; färbende Pigmente; Entschäumungs- und Antischäumungsmittel; Anti-Absetzungs-, Anti-Ablauf- und Konsistenzmittel; Anti-Hautbildungsmittel; Anti-Aufschwimmittel (horizontal und vertikal); Fungizide und Antischimmelmittel; Verdickungsmittel oder Koaleszierungsmittel enthält.

**21.** Aufbringen der härtbaren Schutzschicht-Zusammensetzung nach Anspruch 20 auf ein geformtes oder gestaltetes Substrat und anschließendes Härten, um einen beschichteten oder gestalteten Gegenstand zu erhalten.

## Revendications

**1.** Composé de formule :

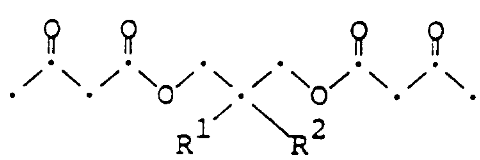,

dans laquelle $R^1$ et $R^2$ sont individuellement alkyle en $C_1$-$C_8$, pourvu que lorsque l'un des restes $R^1$ et $R^2$ est un groupe méthyle, l'autre soit un alkyle en $C_2$-$C_8$.

2. Composé selon la revendication 1, dans lequel $R^1$ est un groupe éthyle et $R^2$ est un groupe n-butyle.

3. Composition d'émail durcissable comprenant
   (1) 15 à 80 % en poids d'un ou plusieurs polyesters et/ou polymères acryliques durcissables ;
   (2) 0 à 50 % en poids d'un solvant approprié ;
   (3) 5 à 40 % en poids d'un agent réticulant ; et
   (4) 5 à 40 % en poids d'un composé, de formule :

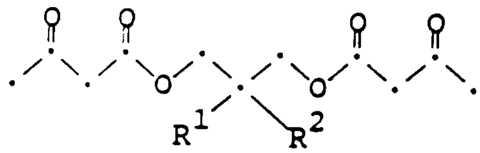 ,

   dans laquelle $R^1$ et $R^2$ sont individuellement alkyle en $C_1$-$C_8$, pourvu que lorsque l'un des restes $R^1$ et $R^2$ est un groupe méthyle, l'autre soit un alkyle en $C_2$-$C_8$.

4. Composition d'émail durcissable selon la revendication 3, dans laquelle l'agent réticulant a deux ou plusieurs groupes de formule :

   $-N(CH_2OR)_2$

   dans laquelle R est groupe alkyle en $C_1$-$C_4$.

5. Composition d'émail durcissable selon la revendication 3, dans laquelle l'agent réticulant est choisi parmi l'hexaméthoxyméthylmélamine, la tétraméthoxyméthylbenzoguanamine, la tétraméthoxyméthylurée et une mélamine butoxy/méthoxy-substituée mixte.

6. Composition d'émail durcissable selon la revendication 3, dans laquelle l'agent réticulant est l'hexaméthoxyméthylmélamine.

7. Composition d'émail durcissable selon la revendication 3, dans laquelle le constituant (1) consiste en un ou plusieurs polyesters réticulables.

8. Composition d'émail durcissable selon la revendication 3, dans laquelle $R^1$ est un groupe éthyle et $R^2$ est un groupe n-butyle.

9. Composition d'émail durcissable comprenant :
   (1) 30 à 70 % en poids d'un ou plusieurs polyesters et/ou polymères acryliques durcissables ;
   (2) 20 à 50% en poids d'un solvant ;
   (3) 5 à 25 % en poids d'un agent réticulant ; et
   (4) 5 à 25 % en poids d'un composé de formule :

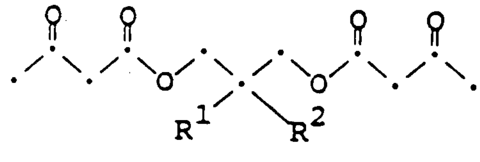 ,

   dans laquelle $R^1$ et $R^2$ sont individuellement alkyle en $C_1$-$C_8$, pourvu que lorsque l'un des restes $R^1$ et $R^2$ est un groupe méthyle, l'autre soit un alkyle en $C_2$-$C_8$.

10. Composition d'émail durcissable selon la revendication 9, dans laquelle l'agent réticulant a deux ou plusieurs groupes de formule :

   $-N(CH_2OR)_2$

dans laquelle R est groupe alkyle en $C_1$-$C_4$.

**11.** Composition d'émail durcissable selon la revendication 9 ou 10, dans laquelle l'agent réticulant est choisi parmi l'hexaméthoxyméthylmélamine, la tétraméthoxyméthylbenzoguanamine, la tétraméthoxyméthylurée et une mélamine butoxy/méthoxy-substituée mixte.

**12.** Composition d'émail durcissable selon la revendication 9 ou 11, dans laquelle l'agent réticulant est l'hexaméthoxyméthylmélamine.

**13.** Composition d'émail durcissable selon la revendication 9, dans laquelle le constituant (1) consiste en un ou plusieurs polyesters réticulables.

**14.** Composition d'émail durcissable selon la revendication 9 ou 13, dans laquelle $R^1$ est un groupe éthyle et $R^2$ est un groupe n-butyle.

**15.** Composition d'émail durcissable comprenant
(1) 45 à 55 % en poids d'un ou plusieurs polyesters et/ou polymères acryliques durcissables ;
(2) 30 à 40 % en poids d'un solvant approprié ;
(3) 7,5 à 12,5 % en poids d'un agent réticulant ; et
(4) 7,5 à 12,5 % en poids d'un composé de formule :

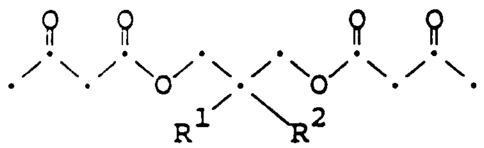

,

dans laquelle $R^1$ et $R^2$ sont individuellement alkyle en $C_1$-$C_8$, pourvu que lorsque l'un des restes $R^1$ et $R^2$ est un groupe méthyle, l'autre soit un alkyle en $C_2$-$C_8$.

**16.** Composition d'émail durcissable selon la revendication 15, dans laquelle l'agent réticulant a deux ou plusieurs groupes de formule :

$-N(CH_2OR)_2$

dans laquelle R est groupe alkyle en $C_1$-$C_4$.

**17.** Composition d'émail durcissable selon la revendication 15 ou 16, dans laquelle l'agent réticulant est choisi parmi l'hexaméthoxyméthylmélamine, la tétraméthoxyméthylbenzoguanamine, la tétraméthoxyméthylurée et une mélamine butoxy/méthoxy-substituée mixte.

**18.** Composition d'émail durcissable selon la revendication 15, dans laquelle l'agent réticulant est l'hexaméthoxyméthylmélamine.

**19.** Composition d'émail durcissable selon la revendication 15, dans laquelle $R^1$ est éthyle et $R^2$ est n-butyle et le constituant (1) consiste en un ou plusieurs polyesters réticulables.

**20.** Composition d'émail durcissable selon la revendication 3, 9 ou 15, comprenant en outre un ou plusieurs agents d'étalement et de contrôle d'écoulement ; des agents mouillants et de dispersion des pigments ; des catalyseurs de réticulation ; des pigments de nuançage ; des agents désémulsifiants et antimoussants ; des agents antisédimentation, anticoulures et donnant du corps ; des agents antipeau ; des agents antinoyage et antiflottation ; des fongicides et agents antimildiou ; des agents épaississants ; ou des agents de coalescence.

**21.** Application des compositions d'émaux réticulables selon la revendication 20 sur un substrat moulé ou façonné et réticulation ultérieure afin d'obtenir un article moulé ou façonné revêtu.